# EUROPEAN PATENT APPLICATION

(11) **EP 3 332 997 A1**
(43) Date of publication of application: **13.06.2018**
(21) Application number: 17203863.0
(22) Date of filing: 27.11.2017
(51) Int. Cl.: B60H 3/00, B60H 1/24, A61L 9/22, F24F 3/16

(54) **VEHICLE INCLUDING STEERING STABILIZING IONIZER**

(30) Priority: 09.12.2016 JP 2016239836
(71) Applicant: TOYOTA JIDOSHA KABUSHIKI KAISHA, Toyota-shi, Aichi-ken, 471-8571 (JP)
(72) Inventor: SETOGUCHI, Daisuke, Aichi-ken,, 471-8571 (JP); KATO, Yasushi, Aichi-ken,, 471-8571 (JP); YAMADA, Koshi, Aichi-ken,, 471-8571 (JP)
(74) Representative: J A Kemp

(57) **Abstract**

A vehicle (12) includes a blowing machine (30) that is disposed on the vehicle (12), a duct (50) having a blow-out port that faces an outer plate, which constitutes a vehicle body surface of the vehicle (12), from a vehicle inside, and an ionizer (40) configured to supply oxide ions into the duct (50). The duct (50) is configured to guide air, which is blown from the blowing machine (30), to the blow-out port.

## Description

### BACKGROUND OF THE INVENTION

### 1. Field of Invention

The present invention relates to a vehicle including a steering stabilizing ionizer that emits oxide ions (O²") toward an outer plate of a vehicle.

### 2. Description of Related Art

A vehicle described in Japanese Unexamined Patent Application Publication No. 2016-168951 (JP 2016-168951 A) includes a charge control device for suppressing splitting of an air flow from a vehicle body surface during traveling and improving steering stability. The charge control device is constituted of an ionizer that generates negative ions, and a pipe coupled to the ionizer, and emits negative ions toward a specific site where an air flow starts to change to a flow away from the vehicle body surface. Accordingly, by reducing a positive charge of the specific site or charging the specific site with a negative charge, generation of a repulsive force between the positively charged air flow and the vehicle body surface can be suppressed, or a Coulomb force is exerted on the air flow, so that the air flow can be suctioned to the vehicle body surface. Additionally, in the charge control device, a configuration in which negative ions are emitted to an inner surface of a vehicle body in the specific site is provided, so that splitting of the air flow can be suppressed without changing the shape of the vehicle body surface.

### SUMMARY OF THE INVENTION

However, since the charge control device (steering stabilizing ionizer device) of the above configuration is solely a configuration in which the pipe is coupled to the ionizer, there is room for improvements from a viewpoint of expanding an emission region of the negative ions to have higher contribution to the steering stability than before. Additionally, in a case where the steering stabilizing ionizer devices is mounted on an actual vehicle, the mounting location poses a problem. That is, the mounting location of the steering stabilizing ionizer device needs that not only the effect of improving the steering stability is higher than before, but also it is easy to secure the durability (water resistance, weather resistance, or the like) of electrical components, and influence on the space and the design of a vehicle cabin is less than before.

For example, as illustrated in FIG. 3 of JP 2016-168951 A, in a case where the steering stabilizing ionizer device is mounted on a tip side (front end side) of a roof, the effect of improving the steering stability is higher than before, and it is easy to secure the durability of the device. However, in order to secure the mounting space of the device, countermeasures, such as swelling a front end of an inner panel (roof headlining) to the vehicle cabin side, are needed. As a result, the space of the vehicle cabin may be narrowed or the design of the vehicle cabin may be restricted.

The invention provides a vehicle including a steering stabilizing ionizer that can enhance the effect of improving steering stability to be higher than before, can easily secure the durability of electrical components, and can have less influence on the space and the design of a vehicle cabin than before.

An aspect of the invention relates to a vehicle including a blowing machine that is disposed on the vehicle, a duct having a blow-out port that faces an outer plate, which constitutes a vehicle body surface of the vehicle, from a vehicle inside, and an ionizer configured to supply oxide ions into the duct. The duct is configured to guide air, which is blown from the blowing machine, to the blow-out port.

In the vehicle according to the aspect of the invention, the blowing machine and the ionizer may be disposed within an instrument panel of the vehicle.

According to the aspect of the invention, the oxide ions (O²⁻) supplied into the duct by the ionizer disposed within the instrument panel of the vehicle is guided to the blow-out port of the duct together with the air blown from the blowing machine disposed within the instrument panel of the vehicle, and is emitted from the blow-out port. Since the blow-out port faces the outer plate, which constitutes the vehicle body surface of the vehicle, from the vehicle inside, the emitted oxide ions can be supplied to the outer plate. Accordingly, a positive charge of the outer plate can be reduced, or the outer plate can be charged with a negative charge. As a result, generation of a repulsive force between a positively charged air flow (traveling wind) and the vehicle body surface can be suppressed, or a Coulomb force is exerted on the air flow, so that the air flow can be suctioned to the vehicle body surface.

Here, since a place where the air flow is relatively easily split from the vehicle body surface is present around the instrument panel (a front end of the vehicle cabin), oxide ions can be emitted toward the outer plate in the above place by disposing the blowing machine and the ionizer within the instrument panel. Moreover, since the oxide ions are emitted together with the air blown from the blowing machine, an emission region for the oxide ions can be expanded. From the above, the effect of improving the steering stability of the vehicle can be made higher than before. Additionally, since the blowing machine and the ionizer are disposed within the instrument panel of the vehicle, securement of the durability of electrical components of the blowing machine and the ionizer is easy. Additionally, compared to a case where the blowing machine and the ionizer are disposed on a roof or the like, influence on the space and the design of the vehicle cabin can be made less than before.

In the vehicle according to the aspect of the invention, the blow-out port may include a front blow-out port that faces a windshield that is the outer plate.

According to the aspect of the invention, the oxide ions are emitted from the front blow-out port toward the windshield. Accordingly, since the positive charge of the windshield can be reduced or the windshield can be charged with the negative charge, splitting of a boundary layer of an air flow that flows along the windshield can be suppressed. As a result, for example, a downward force acting on a front wheel of the vehicle increases, and thus the grounding property of the front wheel is higher than usual, and rolling of the vehicle is suppressed.

In the vehicle according to the aspect of the invention, the blow-out port may include a side blow-out port that faces a front side glass that is the outer plate.

According to the aspect of the invention, the oxide ions are emitted from the side blow-out port toward the front side glass. Accordingly, since the positive charge of the front side glass can be reduced or the front side glass can be charged with the negative charge, splitting of a boundary layer of an air flow that flows along the front side glass can be suppressed. As a result, for example, the stability (straight-running property) of the vehicle in a yaw direction is higher than usual.

The vehicle according to the aspect of the invention may further include a vehicular air-conditioner that is disposed within the instrument panel. The vehicular air-conditioner may include an air-conditioning unit including a blower, a center defroster duct, and a side defroster duct. The blowing machine may be the blower. The duct may be at least one of the center defroster duct and the side defroster duct.

According to the aspect of the invention, some parts of the vehicular air-conditioner that is already installed in the vehicle are shared as constituent elements of the steering stabilizing ionizer device. Accordingly, it is possible to provide the steering stabilizing ionizer device for improving the steering stability at extremely low costs.

In the vehicle according to the aspect of the invention, the duct may be both the center defroster duct and the side defroster duct. The ionizer may be disposed at a branching part from which the center defroster duct and the side defroster duct branch.

According to the aspect of the invention, the ionizer is disposed at the branching part from which the center defroster duct and the side defroster duct branch. Accordingly, the oxide ions generated from one ionizer can be distributed into the respective defroster ducts. As a result, for example, compared to a configuration in which the same number of ionizers as that of each of the defroster ducts are is disposed, less number of ionizers can be made than before. Additionally, since the ionizer is disposed at the branching part, the ionizer for distributing the oxide ions into the respective defroster ducts can be disposed as close as possible to the blow-out ports of the respective defroster ducts. Accordingly, a decrease in the emission amount of the oxide ions from the blow-out ports of the respective defroster ducts can be suppressed.

In the vehicle according to the aspect of the invention, the vehicular air-conditioner may include a control device. The control device may be configured to control operation of the ionizer. The control device may be configured to control a blow-out port mode of the vehicular air-conditioner to a defroster mode and operate the blower and the ionizer when an occupant is turning off air-conditioning performed by the vehicular air-conditioner.

According to the aspect of the invention, when the occupant is turning off the air-conditioning by the vehicular air-conditioner, oxide ions continues being emitted from one or both of the blow-out port of the center defroster and the blow-out port of the side defroster toward one or both of the windshield and the front side glass. Accordingly, even when the air-conditioning is turned off, a blow-out wind can be prevented from hitting an occupant's face while the steering stability is improved. In addition, in order to secure an occupant's comfort, it is preferable to operate the blower at a minimally needed rotational speed.

In the vehicle according to the aspect of the invention, the vehicular air-conditioner may include a display that displays an operational situation of the air-conditioning unit. The control device may be configured to control the display to a state of indicating that the air-conditioning is turned off when the occupant is turning off the air-conditioning,

According to the aspect of the invention, when the air-conditioning performed by the vehicular air-conditioner is turned off, the occupant can be prevented from erroneously recognizing that the air-conditioning is turned on.

In the vehicle according to the aspect of the invention, an electrostatic induction member configured to attract the oxide ions through electrostatic induction may be disposed in the blow-out port.

According to the aspect of the invention, it is estimated that the emission amount of the oxide ions from the blow-out port can be increased by the oxide ions supplied into the duct being attracted to the electrostatic induction member disposed in the blow-out port.

As described above, with the vehicle including the steering stabilizing ionizer according to the aspect of the invention, the effect of improving the steering stability can be made higher than before, and securement of the durability of the electrical components is easy. Moreover, influence on the space and the design of the vehicle cabin can be made less than before.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
FIG. 1 is a perspective view illustrating the configuration of a vehicle cabin front part of a vehicle on which a steering stabilizing ionizer device related to a first embodiment of the invention is mounted when viewed from a vehicle rear side;
FIG. 2 is a schematic view of the steering stabilizing ionizer device related to the first embodiment;
FIG. 3 is a perspective view of the steering stabilizing ionizer device related to the first embodiment;
FIG. 4 is a sectional view illustrating the configuration of the vicinity of a front blow-out port of a duct when viewed from a vehicle left side;
FIG. 5 is a sectional view illustrating the configuration of the vicinity of a side blow-out port of the duct when viewed from the vehicle rear side;
FIG. 6 is a side view of a vehicle related to the first embodiment, and is a view for describing an air flow (traveling wind) that flows on an upper surface side of the vehicle;
FIG. 7 is a plan view of a vehicle related to the first embodiment, and is a view for describing an air flow (traveling wind) that flows on a lateral surface side of the vehicle;
FIG. 8 is a partial sectional view illustrating a vehicular air-conditioner, which is configured to include a steering stabilizing ionizer device related to a second embodiment of the invention when viewed from the vehicle rear side;
FIG. 9 is an enlarged sectional view illustrating a cutting plane taken along line IX-IX of FIG. 8;
FIG. 10 is a system block diagram of an air-conditioning controller in the vehicular air-conditioner related to the second embodiment; and
FIG. 11 is a flowchart illustrating a control in a case where an occupant turns off air-conditioning in the vehicular air-conditioner related to the second embodiment.

### DETAILED DESCRIPTION OF EMBODIMENTS

### First Embodiment

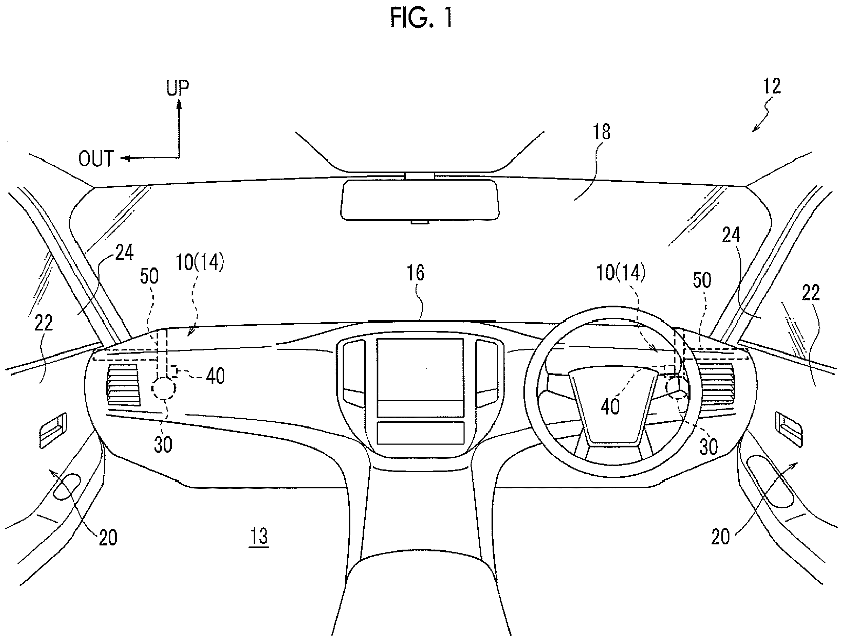

Hereinafter, a steering stabilizing ionizer device 10 and a vehicle 12 related to a first embodiment will be described with reference to FIGS. 1 to 7. In addition, arrow FR, arrow UP, and arrow OUT that are appropriately written in the respective drawings indicate a forward direction (traveling direction) of the vehicle, an upward direction, and the outside in a vehicle width direction, respectively. In the following, in a case where description will be made using a forward-rearward direction, a rightward-leftward direction, and an upward-downward direction, unless otherwise noted, these directions respectively indicate front and rear in a vehicle forward-rearward direction, right and left in a vehicle rightward-leftward direction (vehicle width direction), and up and down in a vehicle upward-downward direction.

### Configuration

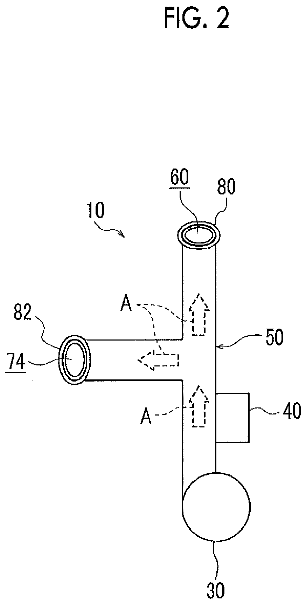

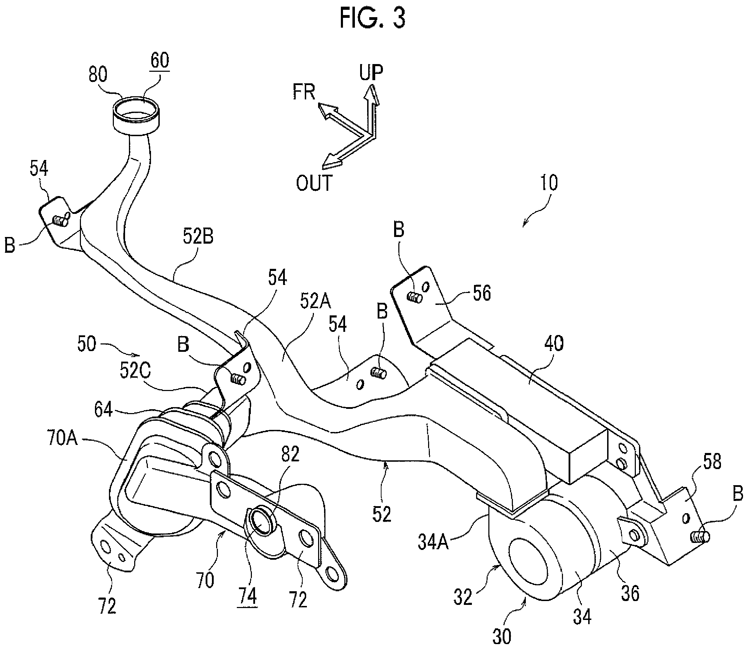

As illustrated in FIGS. 1 to 3, a vehicular ionizer system 14 for improving steering stability is mounted on the vehicle 12 related to the present embodiment. The vehicular ionizer system 14 is constituted of a pair of right and left steering stabilizing ionizer devices (steering stabilizing ionizer devices) 10. The right and left steering stabilizing ionizer devices 10 are disposed on both end sides, in a vehicle width direction, of an instrument panel 16 that is provided at a front end of a vehicle cabin 13, and is provided separately from a vehicular air-conditioner (not illustrated) that is provided within the instrument panel 16. In addition, in FIGS. 1 and 2, the right and left steering stabilizing ionizer devices 10 are schematically illustrated. Additionally, a steering stabilizing ionizer device 10 disposed on a vehicle left side is illustrated in FIG. 3. Since the right and left steering stabilizing ionizer devices 10 basically have the same configuration except for being symmetrically configured, the configuration of the vehicle left side will mainly be described below.

Principal parts (most) of the right and left steering stabilizing ionizer devices 10 are disposed inside the instrument panel 16, and some parts of the ionizer devices are disposed inside door trims 22 in right and left front side doors 20. Specifically, each steering stabilizing ionizer device 10 includes a blowing machine 30 and an ionizer (negative ion generator) 40 that are disposed within the instrument panel 16, a duct 50 that is disposed over the inside of the instrument panel 16 and the inside of the door trim 22, and electrostatic induction rings 80, 82 (not illustrated in FIG. 1) serving as electrostatic induction members attached to the duct 50.

As illustrated in FIG. 3, the blowing machine 30 includes a fan 32 (here, a sirocco fan), and a motor 36 that rotates the fan 32. The duct 50 includes an instrument panel duct 52 that is disposed within the instrument panel 16, and a door trim duct 70 that is disposed within the door trim 22 of a front side door 20. The instrument panel duct 52 and the door trim duct 70 are formed of, for example, resin.

The instrument panel duct 52 is formed in an elongated tubular shape having a vehicle forward-rearward direction as a longitudinal direction as a whole. The instrument panel duct 52 is provided with a plurality of fixing brackets 54, and the instrument panel duct 52 is fixed to the instrument panel 16 using fasteners B (bolts or the like) that pass through the fixing brackets 54. An air blow-out part 34A provided in a casing 34 of the fan 32 is connected to a base end (rear end) of the instrument panel duct 52, and the air blown from the blowing machine 30 flows into the instrument panel duct 52. In addition, in FIG. 2, arrow A indicates a flow direction of the blown air within the duct 50.

Additionally, the ionizer 40 is attached to a base end side of the instrument panel duct 52. The ionizer 40 is formed in the shape of an elongated rectangular parallelepiped and is disposed with the vehicle forward-rearward direction as the longitudinal direction. The ionizer 40 is fixed to the instrument panel duct 52 in a state where an opening (not illustrated) formed on the base end side of the instrument panel duct 52 is closed. The ionizer 40 is configured to generate oxide ions (O²⁻) to supply the oxide ions into the instrument panel duct 52. The fixing brackets 56, 58 are fixed to the ionizer 40 and the motor 36 of the blowing machine 30 by means, such as bolt fastening. Also, the fixing brackets 56, 58 are fixed to the instrument panel 16 using bolts B that pass through the fixing brackets 56, 58. Accordingly, the ionizer 40 and the blowing machine 30 are supported by the instrument panel 16 via the fixing brackets 56, 58.

A branching part 52A is formed at an intermediate part of the instrument panel duct 52 in the longitudinal direction nearer a tip side of the instrument panel duct 52 than the ionizer 40. Also, in the branching part 52A, the instrument panel duct 52 branches into two parts of a front-side extending part 52B and a door-side extending part 52C. For this reason, the blown air that has flowed into the base end side within the instrument panel duct 52 is distributed into the front-side extending part 52B and the door-side extending part 52C.

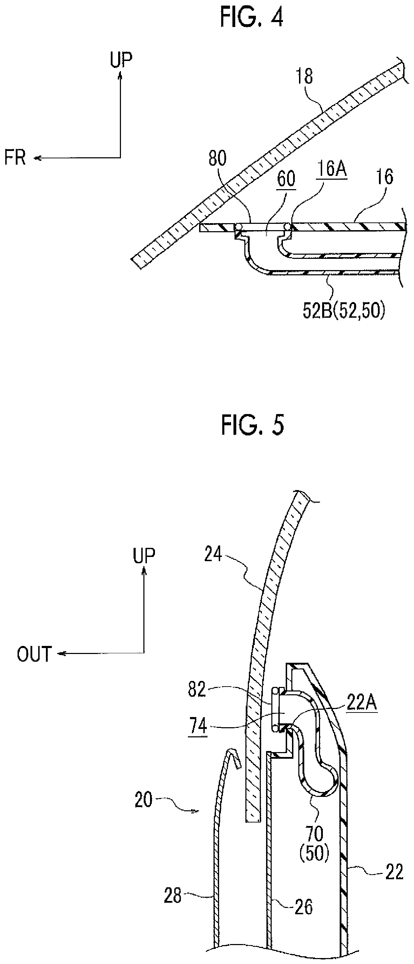

The front-side extending part 52B extends from the branching part 52A to the vehicle front side, and is bent inward in the vehicle width direction at an intermediate part in the longitudinal direction, and a tip thereof is bent to a vehicle upper side. A front blow-out port 60 opening to the vehicle upper side is formed at the tip of the front-side extending part 52B. As illustrated in FIG. 4, the front blow-out port 60 is exposed to the outside of the instrument panel 16 via an opening 16A formed in an upper surface of an instrument panel 16, and approaches and faces a lower end of a windshield (front windshield) 18 from a vehicle lower side. For this reason, the blown air that has flowed into the front-side extending part 52B is blown out from the front blow-out port 60 toward the windshield 18 (blown against the windshield 18). The electrostatic induction ring 80 formed in a ring shape, for example, using metal, such as iron, is coaxially attached to an opening edge of the front blow-out port 60. The electrostatic induction ring 80 is configured to attract oxide ions through electrostatic induction.

In addition, in the present embodiment, the shortest distance between the front blow-out port 60 and the windshield 18 is set to, for example, 20 mm or less. Additionally, the blow-out wind blown out from the front blow-out port 60 is blown against the surface of the windshield 18 on a vehicle cabin side at an angle of 45 degrees or more and 90 degrees or less.

The door-side extending part 52C extends from the branching part 52A to the outside in the vehicle width direction. A boot 64 formed in an annular body (a frame shape) using an elastic substance, such as rubber is mounted on a tip of the door-side extending part 52C. The boot 64 is fitted into an opening (not illustrated) formed on a lateral surface (an outer surface in the vehicle width direction) of the instrument panel 16. The boot 64 is disposed so as to face a front end and an upper end in the door trim 22 of the front side door 20 in a closed state of the front side door 20. The boot 64 corresponds to the door trim duct 70.

The door trim duct 70 is disposed within the door trim 22 of the front side door 20 with the vehicle forward-rearward direction as the longitudinal direction. The door trim duct 70 is provided with a plurality of fixing brackets 72, and the door trim duct 70 is fixed to the door trim 22 using fasteners (bolts or the like) (not illustrated) that pass through the fixing brackets 72.

An opening (not illustrated) opening toward the inside in the vehicle width direction is formed at a base end (front end) of the door trim duct 70, and a flange part 70A extending in the vehicle forward-rearward direction and the vehicle upward-downward direction is formed at an edge of the opening. The flange part 70A comes into close contact with the boot 64 in a closed state of the front side door 20. Accordingly, a gap between the door-side extending part 52C and the door trim duct 70 is sealed by the boot 64, and the inside of the door-side extending part 52C and the inside of the door trim duct 70 are allowed to communicate with each other.

A tip (rear end) of the door trim duct 70 is bent to the outside in the vehicle width direction. A side blow-out port 74 opening to the outside in the vehicle width direction is formed at the tip of the door trim duct 70. As illustrated in FIG. 5, the side blow-out port 74 is exposed to the outside of the door trim 22 via an opening 22A formed at a front end and an upper end of the door trim 22, and approaches and faces the surface of a front side glass 24 on the vehicle cabin side from the inside in the vehicle width direction. For this reason, the blown air that has flowed into the door trim duct 70 from the inside of the door-side extending part 52C is blown out from the side blow-out port 74 toward the front side glass 24 (blown against the front side glass 24). The electrostatic induction ring 82 formed in a ring shape, for example, using metal, such as iron, is coaxially attached to the tip of the door trim duct 70. The electrostatic induction ring 82 is configured to attract oxide ions through electrostatic induction, like the electrostatic induction ring 80.

In addition, in FIG. 5, an inner panel of the front side door 20 is denoted by reference sign 26, and an outer panel of the front side door 20 is denoted by reference sign 28. Additionally, in the present embodiment, the shortest distance between the side blow-out port 74 and the front side glass 24 is set to, for example, 20 mm or less. Additionally, the blow-out wind blown out from the side blow-out port 74 is blown against the surface of the front side glass 24 on the vehicle cabin side at an angle of 45 degrees or more and 90 degrees or less.

In the steering stabilizing ionizer device 10 of the above configuration, for example, the blowing machine 30 and the ionizer 40 are operated (energized) in a state where an ignition switch of the vehicle 12 is turned on.

### Working and Effects

Next, the working and the effects of the first embodiment will be described.

In the steering stabilizing ionizer device 10 provided in the vehicle 12 of the above configuration, the oxide ions (O²⁻) supplied into the duct 50 by the ionizer 40 disposed within the instrument panel 16 are guided to the front blow-out port 60 and the side blow-out port 74 of the duct 50 together with the air blown from the blowing machine 30 disposed within the instrument panel 16, and are emitted from the blow-out ports 60, 74. Since the blow-out ports 60, 74 face outer plates (the windshield 18 and the front side glass 24), which constitutes a vehicle body surface of the vehicle 12, from a vehicle inside, a positive charge on the outer plates can be reduced (removed) or the outer plates can be charged with a negative charge. As a result, generation of a repulsive force between the positively charged air flow (traveling wind) and the vehicle body surface can be suppressed, or a Coulomb force is exerted on the air flow so that the air flow can be suctioned to the vehicle body surface.

Here, since a place where the traveling wind (air flow) is relatively easily split from the vehicle body surface is present around the instrument panel 16 (the front end of the vehicle cabin 13), the oxide ions can be emitted toward the outer plates in the above place by disposing the blowing machine 30 and the ionizer 40 within the instrument panel 16. Moreover, since the oxide ions are emitted together with the air blown from the blowing machine 30, an emission region for the oxide ions can be expanded. From the above, the effect of improving the steering stability of the vehicle 12 can be made higher than before.

The above steering stability improving effect is confirmed by a driving test that the inventors of the present application have executed after mounting the same steering stabilizing ionizer device as the steering stabilizing ionizer device 10 related to the present embodiment on an actual vehicle. In addition, in a case where an ionizer that generates nano ions or a plasma cluster system that generates ions having positive and negative charges is used instead of the ionizer 40 that generates oxide ions (O²⁻), the effect of reducing the positive charge of the outer plates of the vehicle or charging the outer plate with the negative charge is not obtained. It is believed that the reason is because, for example in the case of nano ions, the ions are surrounded by moisture. This point is also confirmed by the test that the inventors of the present application have executed.

Additionally, in the present embodiment, the blowing machine 30 and the ionizer 40 are disposed within the instrument panel 16 (within the vehicle cabin 13), it is easy to secure the durability of electrical components of the blowing machine 30 and the ionizer 40 (a countermeasure for securing water resistance and weather resistance is not needed). That is, in a case where the blowing machine 30 and the ionizer 40 are disposed in a severe disturbance environment outside the vehicle cabin 13, a countermeasure for securing the water resistance and weather resistance the electrical components of the blowing machine 30 and the ionizer 40 is needed. As a result, the cost for mounting the steering stabilizing ionizer device 10 on the vehicle 12 increases markedly. In this point, in the present embodiment, since a countermeasure for securing the water resistance and weather resistance of the electrical components is not needed, the mounting cost of the steering stabilizing ionizer device 10 can be markedly reduced.

Moreover, compared to a case where the blowing machine 30 and the ionizer 40 are disposed within a roof or the like, influence on the space and the design of the vehicle cabin 13 can be made less than before. Particularly, in a case where the steering stabilizing ionizer device 10 includes the blowing machine 30 as in the present embodiment, it is difficult to secure an arrangement space within a roof or the like. However, it is easy to secure the arrangement space within the instrument panel 16.

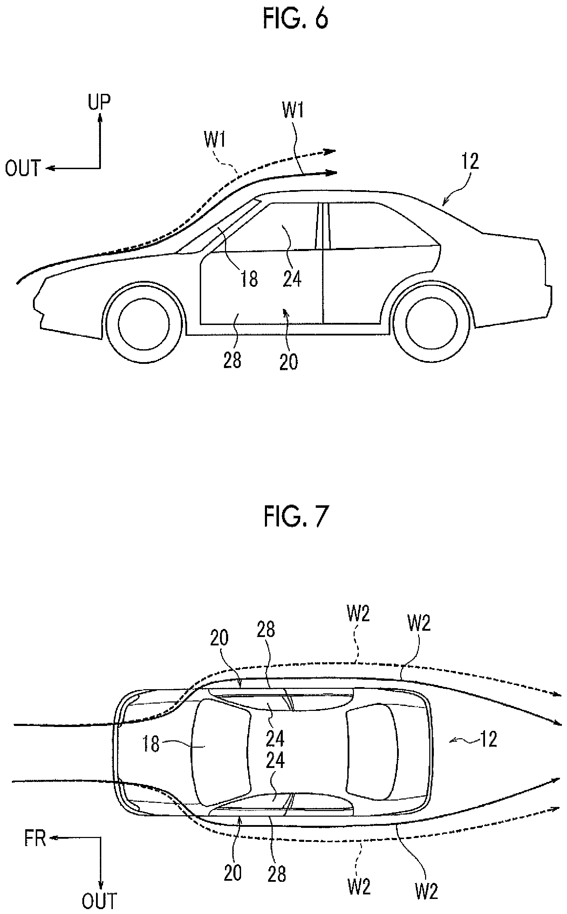

Additionally, in the present embodiment, the duct 50 has the front blow-out port 60 that faces the windshield 18, and oxide ions are emitted from the front blow-out port 60 toward the windshield 18. Accordingly, since the positive charge of the windshield 18 can be reduced or the windshield 18 can be charged with the negative charge, splitting of a boundary layer of an air flow that flows along the windshield 18 can be suppressed (refer to arrow W1 illustrated by a solid line in FIG. 6). As a result, for example, a downward force acting on the front wheel increases, and thus the grounding property of the front wheel is higher than usual, and rolling of the vehicle 12 is suppressed. In addition, in FIG. 6, arrow W1 in a dashed line indicates the flow of an air flow in a case where the vehicle 12 does not include the steering stabilizing ionizer device 10.

Moreover, in the present embodiment, the duct 50 has the side blow-out port 74 that faces the front side glass 24, and oxide ions are emitted from the side blow-out port 74 toward the front side glass 24. Accordingly, since the positive charge of the front side glass 24 can be reduced or the front side glass 24 can be charged with the negative charge, splitting of a boundary layer of an air flow that flows along the front side glass 24 can be suppressed (refer to arrow W2 illustrated by a solid line in FIG. 7). As a result, for example, the stability (straight-running property) of the vehicle 12 in a yaw direction is higher than usual. In addition, in FIG. 7, arrow W2 illustrated by a dashed line indicates the flow of an air flow in a case where the vehicle 12 does not include the steering stabilizing ionizer device 10.

Additionally, in the present embodiment, the electrostatic induction rings 80, 82 that attract oxide ions through electrostatic induction are respectively attached to the opening edges of the front blow-out port 60 and the side blow-out port 74. For this reason, it is estimated that the emission amount of the oxide ions from the blow-out ports 60, 74 can be increased by the oxide ions generated within the duct 50 being attracted to the electrostatic induction rings 80, 82 and guided to the front blow-out port 60 and the side blow-out port 74. In addition, the reason is not certain although the effect is confirmed in the sensory evaluation that the inventors of the present application have executed.

Next, another embodiment of the invention will be described. In addition, the configuration and working that are basically the same as those of the first embodiment will be denoted by the same reference signs as those of the first embodiment, and the description thereof will be omitted.

### Second Embodiment

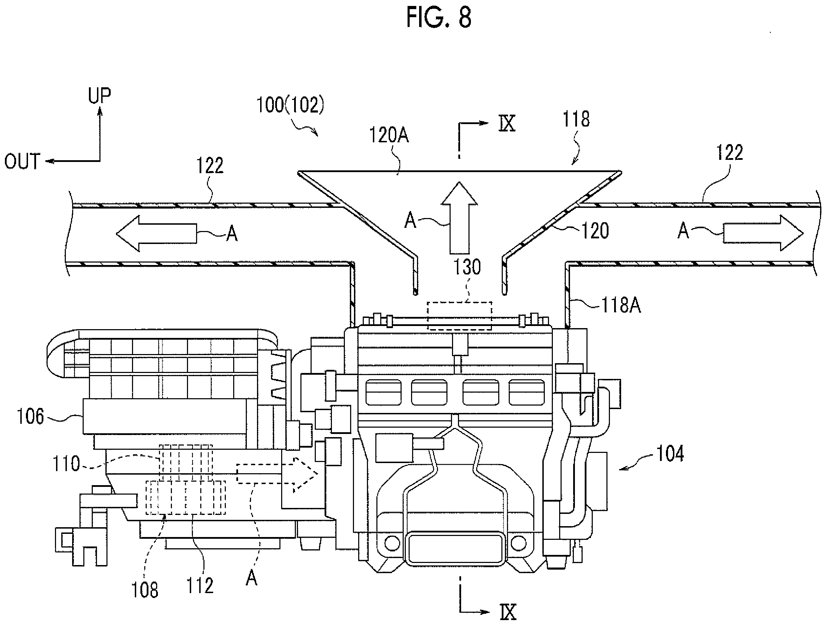

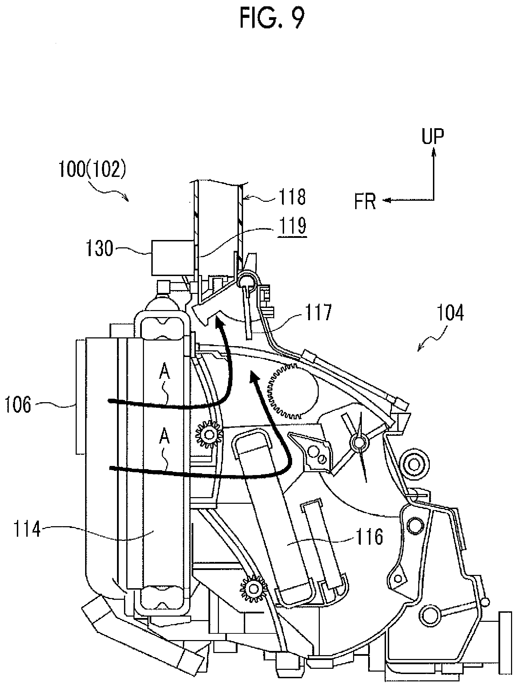

A vehicular air-conditioner 102, which is configured to include a steering stabilizing ionizer device 100 related to a second embodiment, is illustrated in a partial sectional view when viewed from the vehicle rear side in FIG. 8. Additionally, a cutting plane taken along line IX-IX line of FIG. 8 is illustrated in an enlarged sectional view in FIG. 9. The vehicular air-conditioner 102 related to the present embodiment is incorporated into an instrument panel of a vehicle (not illustrated), and includes an air-conditioning unit 104.

A blower 108 including a blower motor 110 and a blower fan 112, an evaporator 114 that cools the air blown by the blower 108, a heater core 116 that reheats the air cooled by the evaporator 114, and the like are provided within a case 106 of the air-conditioning unit 104. Additionally, an upper end of the case 106 has a base end of a defroster duct 118 connected thereto, and is provided with a defroster door 117 that opens and closes the defroster duct 118. The blown air of the blower 108 flows into the defroster duct 118 in a state where the defroster door 117 is open. In addition, in FIGS. 8 and 9, arrow A indicates a flow direction of the blown air within the vehicular air-conditioner 102. Since the air-conditioning unit 104 is of a related art, the detailed description thereof will be omitted.

As illustrated in FIG. 8, a base end of the defroster duct 118 connected to the air-conditioning unit 104 is provided with a branching part 118A. In the branching part 118A, the defroster duct 118 branches into a center defroster duct 120 and a pair of right and left side defroster ducts 122. For this reason, the blown air that has flowed into the base end of the defroster duct 118 flows into the center defroster duct 120 and the right and left side defroster ducts 122, respectively. The center defroster duct 120 has a center defroster blow-out port 120A that faces a windshield (not illustrated) of a vehicle from a vehicle inside. The blown air that has flowed into the center defroster duct 120 is blown out from the center defroster blow-out port toward the windshield. Additionally, the right and left side defroster ducts 122 have side defroster blow-out ports (all are not illustrated) that face front side glasses of the vehicle from the vehicle inside. The blown air that has flowed into the right and left side defroster ducts 122 is blown out from the side defroster blow-out ports toward the front side glasses.

An ionizer (negative ion generator) 130 is attached to the branching part 118A of the defroster duct 118. The ionizer 130 is formed in an elongated rectangular parallelepiped shape having the vehicle width direction as a longitudinal direction. The ionizer 130 is fixed to the defroster duct 118 in a state where an opening 119 formed in the branching part 118A is closed, and is configured to generate oxide ions (O²⁻) to supply the oxide ions into the branching part 118A. The oxide ions supplied into the branching part 118A are distributed into the center defroster duct 120 and the right and left side defroster ducts 122 together with the blown air of the blower 108, and are emitted from the center defroster blow-out port 120A and the side defroster blow-out port. In the present embodiment, the steering stabilizing ionizer device 100 is constituted of the blower 108 of the air-conditioning unit 104, the center defroster duct 120 and the right and left side defroster ducts 122, and the ionizer 130.

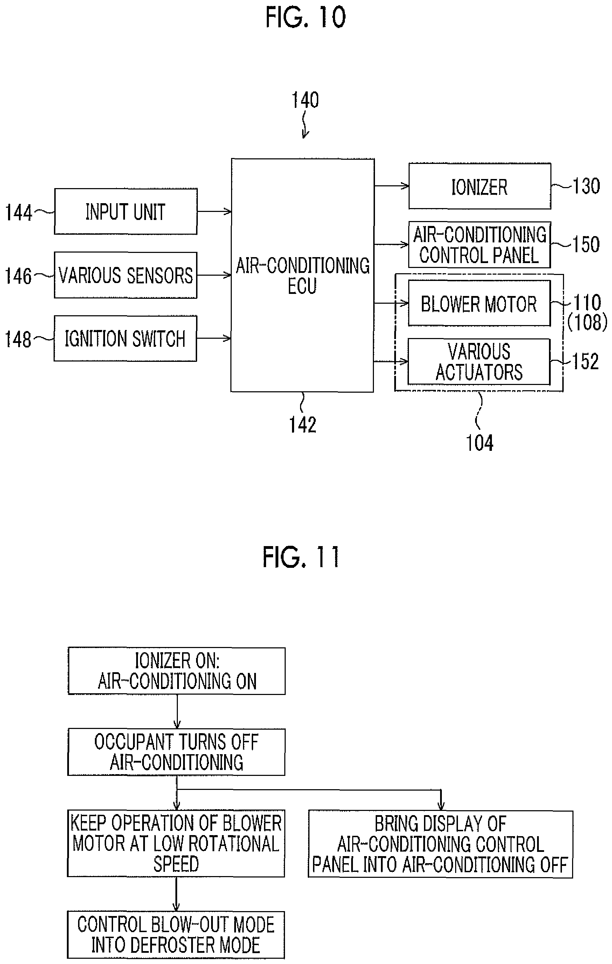

The operation of the air-conditioning unit 104 is controlled by an air-conditioning controller 140 illustrated in FIG. 10. The air-conditioning controller 140 includes an air-conditioning ECU 142 serving as a control device. An input unit 144, various sensors 146, and an ignition switch 148 of the vehicle are electrically connected to the air-conditioning ECU 142. The input unit 144 is provided with various operation switches for performing selection of operation modes of the air-conditioning unit 104, setting of air-conditioning temperature, selection of a blow-out port mode, switching of air volume, and the like, an auto switch for automatically operating the air-conditioning unit 104, and an OFF switch for turning off air-conditioning performed by the air-conditioning unit 104. Additionally, an outside air temperature sensor that measures a temperature outside the vehicle cabin, an inside air temperature sensor that measures a temperature within the vehicle cabin, a solar radiation sensor that measures the quantity of solar radiation, a coolant temperature sensor of an engine outlet, and the like are included in the various sensors 146.

In addition, in the present embodiment, the input unit 144 includes the OFF switch, but the input unit is not limited to this. For example, a configuration may be adopted in which the operation of the air-conditioning unit 104 is stopped by an air volume changeover switch being operated to an OFF position. In that case, the above "state where the air volume changeover switch is operated to the OFF state" becomes a "state where air-conditioning is turned off" in the invention.

Additionally, the ionizer 130 and blower motor 110, and an air-conditioning control panel 150 serving as a display that displays the operational situation of the air-conditioning unit 104 are electrically connected to the air-conditioning ECU 142. Moreover, various actuators 152 for driving various doors including the defroster door 117 provided in the air-conditioning unit 104 are electrically connected to the air-conditioning ECU 142. The air-conditioning ECU 142 is configured to change the blow-out port mode, open and close an intake door, and the like by operating the various actuators 152.

The air-conditioning ECU 142 is configured to always operate the ionizer 130 and the blower 108 in a state where the ignition switch 148 of the vehicle is turned on. That is, not only when an occupant is turning on the air-conditioning of the vehicular air-conditioner 102, but also when the occupant is turning off the air-conditioning of the vehicular air-conditioner 102, the air-conditioning ECU 142 continues operating the ionizer 130 and the blower 108. However, the air-conditioning ECU 142 is configured to operate the blower 108 at a minimally needed rotational speed (at a minimally needed rotational speed capable of emitting oxide ions from the center defroster blow-out port 120A and the side defroster blow-out port) when the occupant is turning off the air-conditioning. Additionally, the air-conditioning ECU 142 changes the display of the air-conditioning control panel 150 to a state where the air-conditioning being turned off is displayed (for example, a state where nothing is displayed on the air-conditioning control panel 150 at all: air-conditioning OFF state) when the occupant is turning off the air-conditioning.

Additionally, the air-conditioning ECU 142 brings the blow-out port mode into a mode selected by the occupant when the occupant is turning on the air-conditioning of the vehicular air-conditioner 102. However, even in a case where the blow-out port mode selected by the occupant is modes except a defroster mode, the defroster door 117 is kept open. In addition, in this case, the air-conditioning ECU 142 is configured to change the opening degree of the defroster door 117 in accordance with an operation mode selected by the occupant.

For example, as illustrated in a flowchart of FIG. 11, the ionizer 130 is operated (turned on) and the occupant is turning on the air-conditioning. Therefore, when the occupant turns off the air-conditioning, the air-conditioning ECU 142 continues operating the blower motor 110 at a minimally needed rotational speed. Additionally, the air-conditioning ECU 142 brings the display of the air-conditioning control panel 150 into an air-conditioning OFF state, and brings the blow-out port mode into the defroster mode.

### Working and Effects

The steering stabilizing ionizer device 100 related to the second embodiment is configured by adding the ionizer 130 to the vehicular air-conditioner 102 that is already installed in the vehicle. Some parts (the blower 108, the center defroster duct 120, and the right and left side defroster ducts 122) of the vehicular air-conditioner 102 are shared as constituent elements of the steering stabilizing ionizer device 100. Accordingly, it is possible to provide the steering stabilizing ionizer device 100 for improving the steering stability at extremely low costs.

Additionally, in the present embodiment, the ionizers 130 is attached to the branching part 118A from which the center defroster duct 120 and the right and left side defroster ducts 122 branch. Accordingly, the oxide ions generated from one ionizer 130 can be distributed into the respective defroster ducts 120, 122. As a result, for example, compared to a configuration in which the same number of ionizers 130 as that of each of the defroster ducts 120, 122 is provided, less number of ionizers 130 can be made than before. Additionally, the ionizer 130 for distributing the oxide ions into the respective defroster ducts 120, 122 can be disposed as close as possible to the blow-out ports (the center defroster blow-out port 120A and the right and left side defroster blow-out ports) of the respective defroster ducts 120, 122. Accordingly, a decrease in the emission amount of the oxide ions from the respective blow-out ports can be suppressed.

Moreover, in the present embodiment, the air-conditioning ECU 142 is configured to control the blow-out port mode of the vehicular air-conditioner 102 to the defroster mode and operate the blower 108 and the ionizer 130 when the occupant is turning off the air-conditioning. Accordingly, even when the air-conditioning is turned off, oxide ions continue being emitted from the center defroster blow-out port 120A and the right and left side defroster blow-out ports toward the windshield and the front side glasses. Accordingly, even when the air-conditioning is turned off, a blow-out wind can be prevented from hitting an occupant's face while the steering stability is improved. Moreover, in this case, since the air-conditioning ECU 142 operates the blower 108 at a minimally needed rotational speed, the occupant's comfort can be secured.

Moreover, in the present embodiment, the air-conditioning ECU 142 is configured to control the air-conditioning control panel 150 to a state of indicating that the air-conditioning is turned off when the occupant is turning off the air-conditioning. Accordingly, when the air-conditioning is turned off, the occupant can be prevented from erroneously recognizing that the air-conditioning is turned on.

### Supplementary Description of Embodiments

In the second embodiment, the air-conditioning controller 140 may be configured to control the air-conditioning unit 104 such that the temperature of the blow-out wind from the center defroster blow-out port 120A and the right and left side defroster blow-out ports become equal to or higher than a predetermined temperature (for example, an outside air temperature). Accordingly, the occurrence of dew condensation on the vehicle inner surfaces of the outer plates (the windshield and each front side glass) against which the blow-out wind is blown can be suppressed.

Additionally, in the first embodiment, a heater and a temperature sensor may be attached to the duct 50, and the temperature of the blow-out wind blown out from the front blow-out port 60 and the side blow-out port 74 may be warmed to a temperature that is equal to or higher than a predetermined temperature. Accordingly, the occurrence of dew condensation on the vehicle inner surfaces of the outer plates (the windshield 18 and each front side glass 24) against which the blow-out wind blown out from the front blow-out port 60 and the side blow-out port 74 is blown can be suppressed.

Additionally, in the first embodiment, the duct 50 is configured to include the front blow-out port 60 that faces the windshield 18, and the side blow-out port 74 that faces each front side glass 24. However, the invention is not limited to this. Either the front blow-out port 60 or the side blow-out port 74 may be omitted. Additionally, the outer plates that face the blow-out ports of the duct may be other than the windshield 18 and each front side glass 24 (for example, an outer panel of an engine hood, an outer panel of an A-pillar, and the like).

Additionally, in the second embodiment, the branching part 118A of the defroster duct 118 is provided with one ionizer 130. However, the invention is not limited to this. For example, ionizers may be disposed on tip sides of the center defroster duct 120 and the right and left side defroster ducts 122, respectively.

Additionally, in the second embodiment, a case where both the center defroster duct 120 and the right and left side defroster ducts 122 are used as ducts has been described. However, the invention is not limited to this, and either the center defroster duct 120 or the right and left side defroster ducts 122 may be used as the ducts. In that case, an ionizer is attached to the one side.

In addition, the invention can be variously changed and carried out without departing from the concept of the invention. Additionally, it goes without saying that the scope of the invention is not limited to the respective embodiments.

## Claims

1. A vehicle comprising:
a blowing machine (30) that is disposed on the vehicle (12);
a duct (50) having a blow-out port that faces an outer plate, which constitutes a vehicle body surface of the vehicle (12), from a vehicle inside, the duct (50) being configured to guide air, which is blown from the blowing machine (30), to the blow-out port; and
an ionizer (40) configured to supply oxide ions into the duct (50).

2. The vehicle according to claim 1, wherein the blowing machine (30) and the ionizer (40) are disposed within an instrument panel (16) of the vehicle (12).

3. The vehicle according to claim 1 or 2, wherein the blow-out port includes a front blow-out port that faces a windshield (18) that is the outer plate.

4. The vehicle according to any one of claims 1 to 3, wherein the blow-out port includes a side blow-out port that faces a front side glass (24) that is the outer plate.

5. The vehicle according to claim 2, further comprising a vehicular air-conditioner that is disposed within the instrument panel (16), wherein:
the vehicular air-conditioner includes an air-conditioning unit including a blower, a center defroster duct (120), and a side defroster duct (122);
the blowing machine is the blower; and
the duct is at least one of the center defroster duct (120) and the side defroster duct (122).

6. The vehicle according to claim 5, wherein:
the duct is both the center defroster duct (120) and the side defroster duct (122); and
the ionizer (130) is disposed at a branching part from which the center defroster duct (120) and the side defroster duct (122) branch.

7. The vehicle according to claim 5 or 6, wherein:
the vehicular air-conditioner includes a control device;
the control device is configured to control operation of the ionizer (130); and
the control device is configured to control a blow-out port mode of the vehicular air-conditioner to a defroster mode and operate the blower and the ionizer (130) when an occupant is turning off air-conditioning performed by the vehicular air-conditioner.

8. The vehicle according to claim 7, wherein:
the vehicular air-conditioner includes a display that displays an operational situation of the air-conditioning unit; and
the control device is configured to control the display to a state of indicating that the air-conditioning is turned off when the occupant is turning off the air-conditioning.

9. The vehicle according to any one of claims 1 to 7, wherein an electrostatic induction member configured to attract the oxide ions through electrostatic induction is disposed in the blow-out port.
